# EUROPÄISCHE PATENTSCHRIFT

(11) **EP 2 183 029 B1**
(45) Veröffentlichungstag und Bekanntmachung des Hinweises auf die Patenterteilung: **27.06.2012**
(21) Anmeldenummer: 08784833.9
(22) Anmeldetag: 17.07.2008
(51) Int. Cl.: A61Q 15/00, A61K 8/365, A61K 8/44, A61K 8/86, A61K 8/20

(54) **STABILISIERUNG KOSMETISCHER ODER DERMATOLOGISCHER FORMULIERUNG MIT MANDELSÄURE**
STABILIZATION OF A COSMETIC OR DERMATOLOGICAL FORMULATION CONTAINING MANDELIC ACID
STABILISATION DE FORMULATION COSMÉTIQUE OU DERMATOLOGIQUE AVEC DE L'ACIDE MANDÉLIQUE

(30) Priorität: 24.07.2007 DE 102070035742
(43) Veröffentlichungstag der Anmeldung: 12.05.2010
(73) Patentinhaber: Beiersdorf AG, 20253 Hamburg (DE)
(72) Erfinder: BIEL, Stefan, 21077 Hamburg (DE); WEINERT, Katrin, 22764 Hamburg (DE)
(74) Vertreter: Hartmann, Jost
(86) Internationale Anmeldenummer: PCT/EP2008/005851
(87) Internationale Veröffentlichungsnummer: WO 2009/012927

(56) Entgegenhaltungen:
- EP-A1- 1 787 629
- WO-A1-2005/105027
- WO-A2-03/039505
- A. NESCI, M. RODRIGUEZ, M. ETCHEVERRY: "Control of Aspergillus growth and aflatoxin production using antioxidants at different conditions of water activity and pH" JOURNAL OF APPLIED MICROBIOLOGY, [Online] Bd. 95, Nr. 2, 11. Juli 2003 (2003-07-11), Seiten 279-287, XP002517216 Gefunden im Internet: URL:10.1046/j.1365-2672.2003.01973.x> [gefunden am 2009-02-26]
- TARA E. GOTTSCHALCK ET AL: "International Cosmetic Ingredient, Dictionary and Handbook, 11th edition, 4 volumen set" 2006, THE COSMETIC, TOILETRY, AND FRAGRANCE ASSOCIATION , USA , XP002517970 ISBN 1-882621-34-4 Monograph 2642 Monograph 2888

## Beschreibung

Die Erfindung betrifft eine kosmetische oder dermatologische Formulierung umfassend Mandelsäure, Antitranspirantien und Hilfsstoffe zu deren verbesserten Stabilisierung.

In der DE 102005017032 werden Mikroemulsionen enthaltend einen Antitranspirantwirkstoff, Mandelsäure und polyethoxylierte O/W-Emulgatoren offenbart.

In der WO 2005105026 werden transparente kosmetische und/oder dermatologische Formulierungen offenbart, die mindestens einen Antitranspirant-Wirkstoff und/oder Deodorant-Wirkstoff, mindestens eine α-Hydroxycarbonsäure und Wasser umfassen. Als Hydroxysäure ist Mandelsäure bevorzugt zu wählen.

Mithilfe der Mandelsäure-Geltechnologie, wie sie in der WO 2005105026 beschrieben ist, ist es erstmals möglich transparente, insbesondere Roll-On-Systeme herzustellen, in die aufgrund der Fließgrenze dieser Formulierungen beispielsweise auch visuelle Effekte wie z.B. Schwebeteilchen, Beads eingearbeitet werden können.

EP 1787629 A1 offenbart kosmetische und/oder dermatologische Formulierung umfassend mindestens einen Antitranspirant-Wirkstoff, Mandelsäure, Wasser und PEG-40 hydrogenated castor oil.

Allerdings zeigt sich, dass diese Mandelsäuresysteme unter besonderen Lagerbedingungen (Lichtlagerung, Lagerung bei 40°C) mitunter nicht lange stabil bleiben, ohne dass Ausfällungen oder Kristallisationen auftreten können.

Wünschenswert ist es daher Mandelsäuregele, insbesondere transparente und insbesondere als Antitranspirant- oder Deodorantprodukte, bereit zu stellen, die eine verbesserte Stabilisierung bei Sonnenbestrahlung und/oder erhöhter Temperatur aufweisen.

Die vorliegende Erfindung umfasst eine kosmetische Formulierung umfassend mindestens einen Antitranspirantwirkstoff, Mandelsäure, Wasser , Magnesiumchlorid und/oder Calciumchlorid und einen oder mehrere Hilfsstoffe.

Die als Hilfsstoffe benannten Verbindungen werden gewählt aus
a.) polyethoxylierte hydrogenierte Rizinusöle, PEG-40 hydrogenated castor oil, PEG-60 hydrogenated castor oil,
b.) phenolische Antioxidantien, wie beispielsweise die Handelsprodukte Tinogard TS, Tinogard TT, Tinogard NOA der Fa.Ciba und/oder
c.) ein oder mehrere Disulfite, insbesondere Natriumdisulfit.

Bevorzugt sind polyethoxylierte hydrogenierte Rizinusöle, insbesondere PEG-40 hydrogenated castor oil. PEG-40 hydrogenated castor oil ist als Handelprodukt unter dem Namen Solutor, Eumulgin oder Fancol erhältlich.

Als bevorzugte Beispiele seien PEG-40 hydrogenated castor oil und PEG-60 hydrogenated castor oil erwähnt. PEG-40 hydrogenated castor oil lässt sich zudem vorteilhaft in Kombination mit Parfum einsetzen.

Ausgenommen als Hilfsstoffe sind die in der DE 102005017032 als Emulgatoren genannten Polyethylenglycolglycerinfettsäureester, Polyethylenglycol(15)glyceryllaurat und/oder Glycerylmonostearate.

Parfum ist für viele Kosmetika ein essentieller Bestandteil, aber er führt häufig zu Problemen und Instabilitäten.

Mit dem oder den Hilfsstoffen, insbesondere dem Hilfsstoff oder Lösevermittler Solutor (PEG-40), lassen sich Parfüme erfindungsgemäß in das Mandelsäure-Gel einarbeiten ohne dass es trüb oder instabil wird. D.h. es ist weiterhin eine vorteilhafte Transparenz und Stabilität von Mandelsäure Gelen, wie sie in der WO 2005105026 beschrieben sind, gegeben.

Gebräuchliche Anteile an Hilfsstoffen liegen vorteilhaft im Bereich von 0,1 bis 10 Gew.%. insbesondere etwa 4 bis 7 Gew.%, bevorzugt im Bereich von 5 bis 6 Gew.%, jeweils bezogen auf die Gesamtmasse der Zubereitung.

Als bevorzugte Hilfsstoffe können daher Lösevermittler, insbesondere PEG-40 HYDROGENATED CASTOR OIL (Warenbez.: Solutor, Eumulgin, Fancol, Cremophor), die insbesondere in Kombination mit Parfüm und/oder deren Bestandteilen, gewählt werden.

Die Hilfsstoffgruppe der phenolischen Antioxidantien umfasst bevorzugt Pentaerythrityl Tetra-di-t-butyl ' Hydroxyhydrocinnamate (Handelsname Tinogard TT, Ciba), Tetrabutyl Ethylidinebisphenol (Handelsname Tinogard NOA, Ciba) oder Octadecyl Di-t-butyl-4-hydroxyhydrocinnamate (Handelsname Tinogard TS. Ciba).

Als Hilfsstoffe können des Weiteren vorteilhaft Disulfite eingesetzt werden, insbesondere Natriumdisulfit.

Der Zusatz von Natriumdisulfit führt zur Vermeidung von Verfarbungsreaktionen und/oder Bildung von Fehlgeruch,

Fehlgerüche und Verfärbungen bilden sich durch ungewollte Oxidationsprozesse. Angeregt werden diese z.B. durch Licht und Warme. Im Falle der Mandelsäure kann mitunter während der Lagerung Benzaldehyd entstehen. Durch Zusatz der Hilfsstoffe, insbesondere von Disulfiten, insbesondere Natriumdisulfit oder Kombinationen von EDTA und Natriumdisulfit, werden diese Oxidationsprozesse unterdrückt und somit Verfärbung und Fehlgeruchsausbildung minimiert. Der Anteil an Bisulfiten liegt vorteilhaft bei etwa 0,1 bis 0.2 Gew.%.

In Kombination mit EDTA wird EDTA bevorzugt zu etwa 1 Gew.% zugesetzt.

Die efindungsgemäße Formulierung lässt sich zu einer viskosen bis pastösen Formulierung gelieren und ermöglicht die Bereitstellung einer transparenten und wenig klebrigen kosmetischen Zubereitung, insbesondere eine Antitranspirant- bzw. Deodorantzubereitung wie in der WO 2005105026 beschrieben. Zum Unterschied werden durch den Einsatz der Hilfsstoffe die zeitweilig auftretenden Probleme durch Ausfällungen erfindungsgemäß minimiert.

Die Hydroxyphenylessigsäure oder auch Phenylglykolsäure mit der Formel H₅C₆ -CH(OH) -NOCH, C₈H₈O₃ ist bekannt unter dem Namen Mandelsäure. Die Mandelsäure ist gut löslich in Wasser, Alkohol, Ether u. 2-Propanol. Synthetisch erhält man die (±)-, auch D- oder L-Mandelsäure aus Benzaldehyd und Blausäure über das α-Hydroxynitril (Cyanohydrin) und dessen saure Hydrolyse entsprechend Abbildung 1:

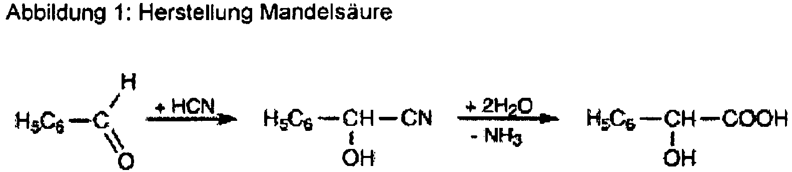

Mittels der Mandelsäure, läßt sich überraschenderweise eine AT- bzw. Deodorantzubereitung aber auch eine beliebige kosmetische Zubereitung herstellen, die die vorteilhaften Eigenschaften, wie Transparenz, geringe Klebrigkeit, Parfumbestandteile und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.

Darüber hinaus trägt Mandelsäure durch seine keratinolytischen Eigenschaften zur Regeneration der Achselhaut bei und wirkt im sauren Milieu der Achsel bakteriostatisch. Aufgrund dieser Eigenschaften ist Mandelsäure hervorragend auch als aktive Komponente in kosmetischen und insbesondere Deo/AT-Produkten geeignet.

Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs-oder drehzahlgesteuerten Viskosimeter erfolgt. Kurze Zeitskalen (schnelle Belastungen) ergeben in der Regel höhere Werte für die Fließgrenze. Eine zu hohe Fließgrenze kann Ursache von Verlaufstörungen sein. Andererseits lässt sich mit geeignet bemessener Fließgrenze die Neigung der flüssigen Formulierung zum Ablaufen unterdrücken.

Die erfindungsgemäße Zubereitung liegt daher vorteilhaft als Gel- bzw. Hydrogel vor und weist ein Fließgrenze auf, wodurch die Ausbringung und Applikation gegenüber den Zubereitungen aus dem Stand der Technik verbessert ist.

Vorzugsweise beträgt der Gesamtanteil an Mandelsäure erfindungsgemäß maximal bis zu 4 Gew.%, vorteilhaft maximal 2,8 Gew.%, bezogen auf die Gesamtmasse einer Zubereitung.

Die erfindungsgemäße Kombination aus AT-Wirkstoff, Mandelsäure, Hilfsstoff und Wasser ermöglicht über einen einzigartigen Verdickungsmechanismus die Herstellung einer insbesondere transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist eine angenehmes Hautgefühl aufgrund der fehlenden Klebrigkeit auf.

Als Antitranspirantwirkstoff lassen sich vorteilhaft saure Aluminium- und/oder Aluminium/Zirkoniumsalze in wässeriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51L
   - Aluminiumsesquichlorhydrat [Al₂(OH)_{4,5}Cl_{1,5}] x H₂O
      Standard Al-Komplexe: Aloxicoll 31 L (Giulini), Westehlor 186 (Westwood Chemicals)
      Aktivierte Al-Komplexe: Reach 301 (Reheis)
   - Aluminiumdichlorhydrat [Al₂(OH)₄Cl₂] x H₂O

Aluminium-ZirKonium-Salze:
- Aluminium/Zirkonium Trichlorhydrex Glycin [Al₄Zr(OH)₁₃Cl₃] x H₂O x Gly Standard Al/Zr-Komplexe: Rezal 33GC (Reheis), AZG-7164 (Summit)
- Aluminium/Zirkonium Tetrachlorhydrex Glycin [Al₄Zr(OH)₁₂Cl₄] x H₂O x Gly
   Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin [Al₈Zr(OH)₂₃Cl₅] x H₂O x Gly
   Standard Al/Zr-Komplexe: Rezal 67 (Reheis), Zirkonal L540. Zirkonal L530 PG (Giulini), Westchlor ZR 80B (Westwood Chemicals)
- Aluminium/Zirkonium Octachlorhydrex Glycin [Al₈Zr(OH)₂₀Cl₈] x H₂O x Gly:
   Westchlor ZR 82B

Ebenso vorteilhaft können aber auch Glycin-freie Aluminium/Zirkonium-Salze eingesetzt werden.

Die Antitranspirant-Wirkstoffe werden in den erfindungsgemäßen Formulierungen in einer Menge von 1 bis 35 Gew.%, vorzugsweise von 1 bis 20 Gew. %, eingesetzt.

Vorteilhaft wird der AT-Wirkstoff aus den Aluminiumsalzen, insbesondere Aluminiumchlorohydrat, aktiviert oder unaktiviert, gewählt.

Vorteilhaft können erfindungsgemaßen Zubereitungen auch Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfume oder deren Parfümbestandteile, Geruchsabsorber. beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Bevorzugt umfasst die erfindungsgemäße Zubereitung ein oder mehrere Parfüme und/oder deren Bestandteile.

Die Menge der Desodorantien (eine oder mehrere Verbindungen) in den Zubereitungen beträgt vorzugsweise 0,01 bis 10 Gew.%, bevorzugt 0,05 bis 5 Gew.% bezogen auf das Gesamtgewicht der Zubereitung.

Dem erfindungsgemäßen System kann weiterhin zusätzlich
- Co-Verdicker, wie beispielsweise Polyvinylpyrrolidone (z.B. Luviskol 30, BASF).
- Polymere enthalten Maleinsäure oder Maleinsäureanhydrid (z.B. Gantrez S- oder Gantrez AN-Typen, ISP International Specialty Products) und/oder
- hydrophob modifzierte Cellulosen (Natrosol 250 HHX Pharm, Hercules)
- Salze, insbesondere bivalente Salze wie beispielsweise Magnesiumchlorid oder Calciumchlorid in Konzentrationen von 0,1% bis 5%, allein oder in Kombination zugeben werden.

Auch diese Co-Verdicker, Polymere bzw. Cellulose und Salze haben einen zusätzlich stabilisierenden Einfluss auf die erfindungsgemäßen Mandelsäuregele.

Der Zusatz von Salzen führt beispielsweise zu einer Erhöhung der Scherstabilität der Mandelsäuregele.

Die bivalenten Neutralsalze Magnesiumchlorid und Calciumchlorid führen zu einer Scherstabilität der erfindungsgemäßen Zubereitungen indem ohne den Zusatz weiße Eintrübungen entstehen können, wenn die Formulierung z.B. stärker geschüttelt wird, die Formel aus einem Roller appliziert wird oder man ggf. zu spät abfüllt. Mit dem Zusatz der Salze passiert das nicht mehr.

Es ist erfindungsgemäß möglich durch den Zusatz ein oder mehrerer Hilfsstoffe die Stabilität von Mandelsäure enthaltenden kosmetischen Zubereitungen zu verbessern. Als Stabilitätsverbesserung ist dabei insbesondere das Vermeiden von Ausfällungen, Eintrübungen oder Kristallisationen, die Stabilisierung bei Sonnenbestrahlung und/oder erhöhter Temperatur, die Vermeidung von Verfärbungsreaktionen und/oder Bildung von Fehlgeruch sowie die Erhöhung der Scherstabilität der Mandelsourezubereitungen zu verstehen.

Die Verwendung von Hilfsstoffen gewählt aus der Gruppe der polyethoxylierte hydrogenierte Rizinusöle, PEG-40 hydrogenated castor oil, PEG-60 hydrogenated castor oil, phenolische Antioxidantien, und/oder Disulfite zur Stabilisierung von Mandelsäure enthaltenden Zubereitungen umfassend Magnesium- und/oder Calciumchlorid ist somit möglich.

Die Stabilisierung ist insbesondere vorteilhaft, wenn wie zuvor erläutert Parfum oder ein oder mehrere derer Bestandteile in der Zubereitung enthalten sind.

Die erfindungsgemäßen kosmetischen und dermatologischen Zubereitungen können kosmetische Zusatzstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, EDTA, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Diese Zusatzstoffe sind von den genannten erfindungsgemäßen Hilfsstoffen zu unterscheiden.

Vorteilhaft werden zur Herstellung erfindungsgemäßer Zubereitungen zunächst ein oder mehrere Hilfsstoffe und ggf. Parfum zusammen gegeben. Der Hilfsstoff und Parfum werden anschließend zu annahemd gleichen Teilen auf die wässrigen Lösungen von Mandelsäure und ACH verteilt, bevor man diese zwecks Vergelung Zusammengibt.

## Patentansprüche

1. Kosmetische und/oder dermatologische Formulierung umfassend mindestens einen Antitranspirant-Wirkstoff, Mandelsäure, Wasser und mindestens einen Hilfsstoff, gewählt aus der Gruppe
- polyethoxylierten hydrogenierten Rizinusöle, PEG-40 hydrogenated castor oil, PEG-60 hydrogenated castor oil,
- phenolische Antioxidantien und/oder
- ein oder mehrere Disulfite, **dadurch gekennzeichnet, dass** die Formulierung Magnesium-und/oder Calciumchlorid umfasst.

2. Formulierung nach Anspruch 1, **dadurch gekennzeichnet, dass** der oder die Hilfsstoffe gewählt werden aus der Gruppe Pentaerythrityl Tetra-di-t-butyl Hydroxyhydrocinnamate, Tetrabutyl Ethylidinebisphenol, Octadecyl Di-t-butyl-4-hydroxyhydrocinnamate und/oder Natriumdisulfit.

3. Formulierung nach Anspruch 1 oder 2, umfassend ein oder mehrere Parfume und/oder deren Bestandteile.

4. Formulierung nach einem der vorstehenden Ansprüche, **dadurch gekennzeichnet, dass** Natriumdisulfit in Kombination mit Ethylendiamintetraessigsäure (EDTA) als Hilfsstoff gewählt wird.

5. Formulierung nach einem der vorstehenden Ansprüche, **dadurch gekennzeichnet, dass** der Antitranspirant-Wirkstoff aus der Gruppe der Aluminium-Salze, bevorzugt Aluminium-Chlorohydrat oder Aluminium-Zirkonium-Salze, gewählt wird.

6. Verwendung von Hilfsstoffen gewählt aus der Gruppe der polyethoxylierten hydrogenierten Rizinusöle, PEG-40 hydrogenated castor oil, PEG-60 hydrogenated castor oil, phenolische Antioxidantien und/oder ein oder mehrere Disulfite zur Stabilisierung von Mandelsäure enthaltenden Zubereitungen, **dadurch gekennzeichnet, dass** die Formulierung Magnesium-und/oder Calciumchlorid umfasst.

7. Verwendung nach Anspruch 6, **dadurch gekennzeichnet, dass** zusätzlich Parfum oder ein oder mehrere derer Bestandteile in der Zubereitung enthalten sind.

## Claims

1. Cosmetic and/or dermatological formulation comprising at least one antiperspirant active ingredient, mandelic acid, water and at least one auxiliary selected from the group
- polyethoxylated hydrogenated castor oils, PEG-40 hydrogenated castor oil, PEG-60 hydrogenated castor oil,
- phenolic antioxidants and/or
- one or more disulphites, **characterized in that** the formulation comprises magnesium chloride and/or calcium chloride.

2. Formulation according to Claim 1, **characterized in that** the auxiliary or auxiliaries are selected from the group pentaerythrityl tetra-di-t-butyl hydroxyhydrocinnamate, tetrabutyl ethylidenebisphenol, octadecyl di-t-butyl-4-hydroxyhydrocinnamate and/or sodium disulphite.

3. Formulation according to Claim 1 or 2, comprising one or more perfumes and/or constituents thereof.

4. Formulation according to one of the preceding claims, **characterized in that** sodium disulphite in combination with ethylenediaminetetraacetic acid (EDTA) is selected as auxiliary.

5. Formulation according to one of the preceding claims, **characterized in that** the antiperspirant active ingredient is selected from the group of the aluminium salts, preferably aluminium chlorohydrate or aluminium-zirconium salts.

6. Use of auxiliaries selected from the group of the polyethoxylated hydrogenated castor oils, PEG-40 hydrogenated castor oil, PEG-60 hydrogenated castor oil, phenolic antioxidants and/or one or more disulphites for stabilizing preparations containing mandelic acid, **characterized in that** the formulation comprises magnesium chloride and/or calcium chloride.

7. Use according to Claim 6, **characterized in that** perfume or one or more of its constituents are additionally present in the preparation.

## Revendications

1. Composition cosmétique et/ou dermatologique comprenant au moins une substance active antisudorale, de l'acide mandélique, de l'eau et au moins un adjuvant, choisi dans le groupe constitué par
- des huiles de ricine polyéthoxylées hydrogénées, PEG-40 hydrogenated castor oil, PEG-60 hydrogenated castor oil,
- des antioxydants phénoliques et/ou
- un ou plusieurs bisulfites, **caractérisée en ce que** la composition comprend du chlorure de magnésium et/ou du chlorure de calcium.

2. Composition selon la revendication 1, **caractérisée en ce que** l'adjuvant ou les adjuvants est/sont choisi(s) dans le groupe constitué par le Pentaerythrityl Tetra-di-t-butyl Hydroxyhydrocinnamate, le Tetrabutyl Ethylidinebisphenol, l'Octadecyl Di-t-butyl-4-hydroxyhydrocinnamate et/ou le bisulfite de sodium.

3. Composition selon la revendication 1 ou 2, comprenant un ou plusieurs parfums et/ou composants de ceux-ci.

4. Composition selon l'une quelconque des revendications précédentes, **caractérisée en ce qu'**on choisit comme adjuvant le bisulfite de sodium en association avec l'acide éthylènediaminetétraacétique (EDTA).

5. Composition selon l'une quelconque des revendications précédentes, **caractérisée en ce qu'**on choisit la substance active antisudorale dans le groupe des sels d'aluminium, de préférence le chlorhydrate d'aluminium ou des sels d'aluminium-zirconium.

6. Utilisation d'adjuvants choisis dans le groupe constitué par des huiles de ricine polyéthoxylées hydrogénées, PEG-40 hydrogenated castor oil, PEG-60 hydrogenated castor oil, des antioxydants phénoliques et/ou un ou plusieurs bisulfites, pour la stabilisation de préparations contenant de l'acide mandélique, **caractérisée en ce que** la composition contient du chlorure de magnésium et/ou du chlorure de calcium.

7. Utilisation selon la revendication 6, **caractérisée en ce qu'**en ouvre un parfum ou un ou plusieurs de ses composants sont contenus dans la préparation.
